# EUROPEAN PATENT APPLICATION

(11) **EP 4 483 949 A1**
(43) Date of publication of application: **01.01.2025**
(21) Application number: 24185659.0
(22) Date of filing: 01.07.2024
(51) Int. Cl.: A61N 5/10

(54) **APPARATUS FOR PHOTON FLASH TREATMENT, METHOD FOR PHOTON BEAM FORMATION, AND COMPUTER READABLE STORAGE MEDIUM**

(30) Priority: 30.06.2023 CN 202310800392; 30.06.2023 CN 202310800422
(71) Applicant: Shanghai United Imaging Healthcare Co., Ltd., Shanghai 201807 (CN)
(72) Inventor: ZHOU, Liuyuan, Shanghai, 201807 (CN); HE, Shoubo, Shanghai, 201807 (CN); WANG, Peng, Shanghai, 201807 (CN); PAN, Gang, Shanghai, 201807 (CN); NI, Cheng, Shanghai, 201807 (CN)
(74) Representative: Viering, Jentschura & Partner mbB Patent- und Rechtsanwälte

(57) **Abstract**

An apparatus (100) for photon flash treatment, a method for photon beam formation, and a computer readable storage medium are provided. The apparatus includes an electron beam emitter (9), a deflection member (30), a deflection mechanism (40), and a radiation delivery device (50). The electron beam emitter (9) is configured to emit an accelerated electron beam. The deflection member (30) can perform first deflection processing on the accelerated electron beam. The deflection mechanism (40) is configured to receive the electron beam after the first deflection processing, and perform second deflection processing on the electron beam after the first deflection processing. The radiation delivery device (50) is configured to form a photon beam by receiving the electron beam after the first deflection processing or after the second deflection processing.

## Description

### CROSS-REFERENCE TO RELATED APPLICATIONS

This application claims priority to Chinese patent applications No. 202310800422.6, filed on June 30, 2023, titled "APPARATUS FOR PHOTON FLASH TREATMENT, METHOD FOR PHOTON BEAM FORMATION, AND COMPUTER READABLE STORAGE MEDIUM", and No. 202310800392.9, filed on June 30, 2023, titled "APPARATUS FOR PHOTON FLASH TREATMENT, METHOD FOR PHOTON BEAM FORMATION, AND COMPUTER READABLE STORAGE MEDIUM".

### TECHNICAL FIELD

The present invention generally relates to the field of medical devices, and in particular, to an apparatus for photon flash treatment, a method for photon beam formation, and a computer readable storage medium.

### BACKGROUND

Flash treatment is a therapeutic solution of radiation treatment for a tumor. A dosage rate of flash treatment is many times higher than that of current radiation treatment, and accurate dosage can be provided.

At present, in a conventional flash treatment solution that uses a photon beam, a superconducting accelerator is generally used to generate the photon beam required for the flash treatment, and then multi-angle exposure to the tumor is implemented by using a rotatable gantry. Although the foregoing solution can generate the photon beam applicable to the flash treatment and implement multi-angle exposure to the tumor, due to a manner of linear acceleration when the superconducting accelerator is in operation, a device to which the solution is applied is not only large in size, but also high in construction costs and subsequent maintenance costs. In addition, in the related art, a rotation of the gantry is used to adjust an emission angle of the photon beam, so as to adapt to different tumor positions of different patients. However, mechanical operation adjustment is inconvenient and adjustment efficiency is low.

### SUMMARY

According to various embodiments of the present invention, an apparatus for photon flash treatment, a method for photon beam formation, and a computer readable storage medium are provided.

In a first aspect, an apparatus for photon flash treatment is provided. The apparatus for photon flash treatment includes an electron beam emitter, a deflection member, a deflection mechanism, and a radiation delivery device. The electron beam emitter is configured to emit an accelerated electron beam. The deflection member is configured to be capable of performing first deflection processing on the accelerated electron beam. The deflection mechanism is configured to perform second deflection processing on the electron beam after the first deflection processing. The radiation delivery device is configured to form a photon beam from the electron beam after the first deflection processing or after the second deflection processing.

In an embodiment, the deflection member is separated from the deflection mechanism by a distance in a planar direction of the deflection mechanism.

In an embodiment, the deflection member is separated from the deflection mechanism by a distance along an axial direction of the deflection mechanism.

In an embodiment, the deflection mechanism is configured to be distributed around a ring.

In an embodiment, the radiation delivery device further includes either or both of a target assembly and a multi-leaf collimator, the target assembly is configured to form the photon beam by receiving the electron beam after the first deflection processing or after the second deflection processing, and the multi-leaf collimator is configured to adjust a beam shape of the photon beam from the target assembly.

In an embodiment, the apparatus for photon flash treatment includes one radiation delivery device capable of moving around a target region. Alternatively, the apparatus for photon flash treatment includes a plurality of radiation delivery devices arranged at intervals around the target region, and at least one of the plurality of radiation delivery devices is fixed or movable relative to the target region.

In an embodiment, the electron beam emitter further includes an electron beam generator and an electron cyclotron. The electron beam generator is configured to generate an electron beam, and the electron cyclotron is configured to accelerate the electron beam.

In an embodiment, the apparatus for photon flash treatment includes two groups of deflection mechanisms, which are arranged at both sides of the target region symmetrically.

In an embodiment, the apparatus for photon flash treatment further includes a beam splitter configured to direct the electron beam to two deflection members through different paths, and the two deflection members are corresponding to the two groups of deflection mechanisms, respectively.

In an embodiment, the electron cyclotron further includes an acceleration cavity and two groups of vector magnets, and the two groups of vector magnets are arranged at both sides of the acceleration cavity.

In an embodiment, the acceleration cavity further includes a waveguide resonant cavity or a coaxial linear resonant cavity.

In an embodiment, the target assembly is distributed in an annular manner.

In an embodiment, energy of the photon beam is in a range of 6MeV to 10MeV.

In an embodiment, the electron cyclotron further includes a lead-out member, the lead-out member is disposed on a cyclic path of the electron beam in the electron cyclotron, the lead-out member is configured to lead out the accelerated electron beam, and a position of the lead-out member is capable of being adjusted relative to the electron cyclotron.

In an embodiment, the electron cyclotron further includes at least one of a betatron, a petal-shaped accelerator, or a race-track microtron.

In an embodiment, a generated magnetic field strength or a generated electric field strength of the deflection member is capable of being adjusted when the deflection member is powered on, so that the electron beam after the first deflection processing is capable of entering the deflection mechanism at different angles.

In an embodiment, the deflection member further includes either or both of a vector magnet and a deflection resonant cavity.

In an embodiment, the apparatus for photon flash treatment further includes a vacuum pump which is disposed in an electron transport path.

In a second aspect, a method for photon beam formation is provided. The method for photon beam formation includes: emitting an accelerated electron beam by an electron beam emitter; performing first deflection processing on the accelerated electron beam by a deflection member, and directing the electron beam after the first deflection processing to a deflection mechanism; performing second deflection processing on the electron beam after the first deflection processing by the deflection mechanism, and directing the electron beam after the second deflection processing to a radiation delivery device; and forming a photon beam via the radiation delivery device by receiving the electron beam after the first deflection processing or after the second deflection processing.

In an embodiment, the electron beam emitter further includes an electron beam generator and an electron cyclotron, and emitting an accelerated electron beam by an electron beam emitter further includes: generating an electron beam by the electron beam generator, and accelerating the electron beam by the electron cyclotron.

In a third aspect, a computer readable storage medium is provided. An instruction is stored on the computer readable storage medium. When executed by a processor, the instruction implements the above method.

Details of one or more embodiments of the present invention are set forth in the following accompanying drawings and description. Other features, objectives, and advantages of the present invention become obvious with reference to the specification, the accompanying drawings, and the claims.

### BRIEF DESCRIPTION OF THE DRAWINGS

In order to more clearly illustrate the technical solutions in the embodiments of the present invention or the related technology, the accompanying drawings to be used in the description of the embodiments or the related technology will be briefly introduced below, and it will be obvious that the accompanying drawings in the following description are only some of the embodiments of the present invention, and that, for one skilled in the art, other accompanying drawings can be obtained based on these accompanying drawings without putting in creative labor.
FIG. 1 is a schematic diagram of an apparatus for photon flash treatment in an embodiment of the present invention.
FIG. 2 is a schematic diagram of an apparatus for photon flash treatment in another embodiment of the present invention.
FIG. 3 is a schematic diagram of an apparatus for photon flash treatment in an embodiment of the present invention.
FIG. 4 is a schematic diagram of an electron beam generator and an electron cyclotron in an embodiment of the present invention.
FIG. 5 is a schematic diagram of a deflection member and a deflection mechanism in an embodiment of the present invention.
FIG. 6 is a schematic diagram of a deflection member in an embodiment of the present invention.
FIG. 7 is a schematic diagram of a deflection mechanism in an embodiment of the present invention.
FIG. 8 is a flowchart of a method for photon beam formation in an embodiment of the present invention.
FIG. 9 is a part flowchart of a method for photon beam formation in an embodiment of the present invention.
FIG. 10 is a schematic diagram of a computer readable storage medium in an embodiment of the present invention.

In the figures, 100 represents an apparatus for photon flash treatment, 9 represents an electron beam emitter, 10 represents an electron beam generator, 20 represents an electron cyclotron, 21 represents an acceleration cavity, 22 represents a vector magnet, 23 represents a lead-out member, 30 represents a deflection member, 40 represents a deflection mechanism, 50 represents a radiation delivery device, 51 represent a target assembly, 52 represent a multi-leaf collimator, 60 represents a beam splitter, 70 represents a bed, and 80 represents a vacuum pump.

### DETAILED DESCRIPTION OF THE EMBODIMENT

The technical solutions in the embodiments of the present invention will be described clearly and completely in the following in conjunction with the accompanying drawings in the embodiments of the present invention, and it is obvious that the described embodiments are only a part of the embodiments of the present invention, but not all of the embodiments. Based on the embodiments in the present invention, all other embodiments obtained by one skilled in the art without making creative labor fall within the scope of protection of the present invention.

It should be noted that when an element is considered to be "disposed on" another element, it can be directly disposed to another element, or there can be a centered element. When an element is considered to be "set on" another element, it can be directly set on another element, or there can be a centered element at the same time. When an element is considered to be "fixed to" another element, it can be directly fixed to another element, or there can be a centered element at the same time.

Unless defined otherwise, technical terms or scientific terms involved in the present invention have the same meanings as would generally understood by one skilled in the technical field of the present invention. The terms used in the specification of the present invention are merely intended to describe specific implementation manners, and are not intended to limit the present invention. The term "and/or" as used herein includes any and all combinations of one or more associated listed items.

In the present invention, an apparatus 100 for photon flash treatment is provided, which is configured to generate a photon beam that is applicable to flash treatment. For example, the photon beam may include X-rays, gamma-rays, and so on. Energy of the photon beam may be in a range of 6 MeV to 10 MeV The energy of the photon beam may be in other ranges, such as in a range of 5 MeV to 12 MeV, 7 MeV to 11 MeV, or higher than 12 MeV Alternatively, the energy of the photon beam may be far greater than that of a normal photon beam. Correspondingly, the photon beam generated by the apparatus 100 for photon flash treatment in the present invention may implement radiation transmission at a high dosage rate. Therefore, a desired radiotherapy may be completed in a very short time. In addition, when a tumor of a patient is irradiated with the photon beam, a requirement for using deep treatment may be implemented. It should be noted that the energy of the photon beam generated when the apparatus 100 for photon flash treatment in the present invention is in operation may be specifically 6 MeV, 7 MeV, 10 MeV, or the like, the energy of the photon beam may be specifically set according to a treatment requirement during flash treatment, which is not described herein.

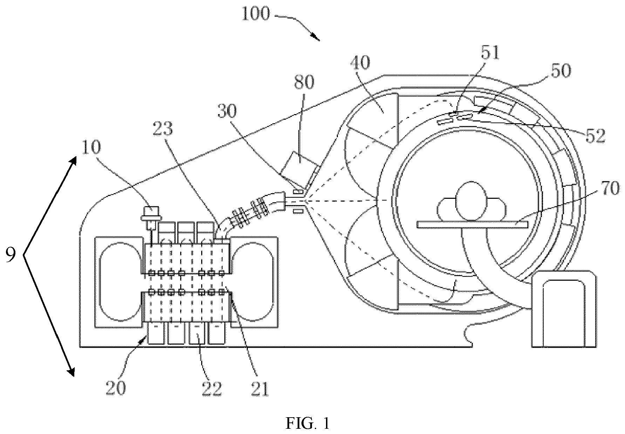

Referring to FIG. 1, an apparatus 100 for photon flash treatment provided in an embodiment of the present invention includes an electron beam emitter 9, a deflection member 30, a deflection mechanism 40, and a radiation delivery device 50. The electron beam emitter 9 is configured to emit an accelerated electron beam. The deflection member 30 is configured to be capable of performing first deflection processing on the accelerated electron beam. The deflection mechanism 40 is configured to receive the electron beam after the first deflection processing, and perform second deflection processing on the electron beam after the first deflection processing. The radiation delivery device 50 is configured to receive the electron beam after the first deflection processing from the deflection member 30 or after the second deflection processing from the deflection mechanism 40, and convert the electron beam into a photon beam. The photon beam may be emitted towards a target region. Alternatively, the deflection member 30 may be disposed on a path of the accelerated electron beam.

In an embodiment, the electron beam emitter 9 may include an electron beam generator 10 and an electron cyclotron 20. The electron beam generator 10 is configured to generate an electron beam. The electron cyclotron 20 is configured to receive the electron beam from the electron beam generator 10, accelerate the electron beam, and lead out the accelerated electron beam.

It may be understood that acceleration of the electron beam is implemented in various manners, such as cyclotron acceleration and detour acceleration, so that a volume required by the electron accelerator to accelerate a photon beam to the photon beam that meets requirement of flash treatment is reduced, and acceleration efficiency of the electron beam is improved, thereby reducing a volume of the apparatus 100 for photon flash treatment and reducing costs. For example, acceleration of the electron beam may be implemented in a cyclotron acceleration manner, so that continuous multi-circle acceleration processing may be performed on the electron beam when the electron cyclotron 20 is in operation. In this way, a land occupation region may be reduced, and acceleration efficiency may be improved. Alternatively, the apparatus 100 for photon flash treatment may control, by controlling intensity of an electric field and/or a magnetic field generated when the deflection member 30 is in operation, the target region to which a final photon beam is converged or delivered. In this way, efficiency and flexibility of adjusting the target region may be improved after the photon beam is converged when the apparatus 100 for photon flash treatment is in operation. For example, the deflection member 30 may include electrically energized plates (plates, arc plates, or arch plates) opposite to each other, an electromagnetic body, a permanent magnet, a combination thereof, or the like. For example, in a case that the deflection member 30 includes electrically energized plates opposite to each other, an electric field strength between the electrically energized plates may be controlled by controlling a current value leading to the deflection member 30. For example, in a case that the deflection member 30 includes an electromagnetic body, a current value leading to the deflection member 30 may be controlled to control a generated magnetic field strength when the deflection member 30 is in operation. In a case that the deflection member 30 includes a permanent magnet, a generated magnetic field strength when the deflection member 30 is in operation may be controlled by a temperature of the deflection member 30 or an external magnetic field.

It should be noted that the target region refers to a location to which the photon beam is expected to be converged or delivered. As a non-limiting example, the target region may be an isocenter region, a specific volume around the isocenter region, or several discrete point positions. As a specific example, the target region may include a region of interest (ROI), for example, a location at which a tumor or another lesion is located in a patient to which the apparatus 100 for photon flash treatment functions when the apparatus 100 for photon flash treatment is in operation, that is, the apparatus 100 for photon flash treatment may accurately irradiate the tumor or the lesion when the apparatus 100 for photon flash treatment operates, and achieve a purpose of the flash treatment. In addition, the target region may also be a region at various other living bodies or non-living bodies. As an example, the target region may be a region on an irradiated experiment (for example, a dosage verification model, a small animal for experiment, a dosage tester), or the like.

In some embodiments, the electron beam generator 10 may be specifically configured as an electron gun, and when the electron gun is in operation, the electron beam may be emitted to the electron cyclotron 20. It may be understood that the electron beam generator 10 is not limited to the electron gun. For one skilled in the art, the electron beam generator 10 may be configured as another apparatus that can generate the electron beam, for example, an electron transmitter, an electron diode, and so on.

Referring to FIG. 1, FIG. 2, and FIG. 4, the electron cyclotron 20 may include an acceleration cavity 21 and two groups of vector magnets 22. The acceleration cavity 21 is capable of performing acceleration processing on the electron beam in the acceleration cavity 21. The two groups of vector magnets 22 may be disposed at both sides of the acceleration cavity 21 and configured to guide the electron beam emitted from the acceleration cavity 21 to re-enter the acceleration cavity 21. As a non-limiting example, when the electron cyclotron 20 is in operation, the electron beam may circulate in the electron cyclotron 20 and pass through the acceleration cavity 21 continuously, and acceleration of the electron beam may be performed by the acceleration cavity 21, so that a circle formed by each circulation path of the electron beam gradually increases. At the same time, energy of the electron beam may be also continuously strengthened. It should be noted that, when the two groups of vector magnets 22 are in operation, deflection processing may be performed on the electron beam, so as to drive the electron beam to turn. It should be noted that the electron beam formed when the electron cyclotron 20 is in operation has a relatively high beam current, so that the electron beam meets a requirement of radiation dosage rate of a flash treatment. It should be noted that the foregoing electron accelerator 20 rotates the electron beam, which means that the electron beam may sequentially pass through the acceleration cavity 21 of the electron accelerator 20 before being led out of the electron accelerator 20. As a non-limiting example, the electron beam may successively accelerate from a same position of the acceleration cavity 21, or may sequentially accelerate from different positions of the acceleration cavity 21, and implement detour rotation acceleration.

As a non-limiting example, the acceleration cavity 21 may be configured as a waveguide resonant cavity or a coaxial linear resonant cavity, so that each time the electron beam passes through the waveguide resonant cavity or the coaxial linear resonant cavity, a specific energy gain may be obtained, and an objective of accelerating the electron beam may be achieved.

The electron cyclotron 20 may further include a lead-out member 23. The lead-out member 23 may be disposed on a cyclic path of the electron beam in the electron cyclotron 20, and may be configured to lead out the accelerated electron beam formed after acceleration from the accelerating cavity 21. For example, the deflection member 30 may include electrically energized plates (plates, arc plates, or arch plates) opposite to each other, an electromagnetic body, a permanent magnet, a combination thereof, or the like. As a non-limiting example, in a case that the electron beam in the electron accelerator 20 travels to the lead-out member 23, the lead-out member 23 may deflect the electron beam away from a previous traveling path by an electric field and/or a magnetic field, so that the electron beam may be led out of the electron accelerator 20.

In some embodiments, a position of the lead-out member 23 is capable of being adjusted relative to the electron cyclotron 20. For example, different positions of the lead-out member 23 in the electron cyclotron 20 are in a one-to-one correspondence with different cyclic paths of the electron beam in the electron cyclotron 20. In this way, different energy choices for the electron cyclotron 20 to derive the electron beam from the lead-out member 23 may be implemented by adjusting the position of the lead-out member 23 in the electron cyclotron 20. In this way, it is convenient for the electron cyclotron 20 to control selection of electron beams with different energy, and meet different requirements of the apparatus 100 for photon flash treatment when in operation.

As a specific example, a mounting channel (not shown in the figures) may be disposed on the electron cyclotron 20. The lead-out member 23 may be specifically disposed in the mounting channel, and may be controlled by a telescopic cylinder or a telescopic motor to adjust the position of the lead-out member 23 in the mounting channel. In this way, control may be implemented for adjusting different positions of the lead-out member 23 in the mounting channel, that is, automatic control may be implemented to adjust the position of the lead-out member 23 in the electron cyclotron 20.

In some embodiments, the electron cyclotron 20 may include at least one of a betatron, a petal-shaped accelerator, or a race-track microtron.

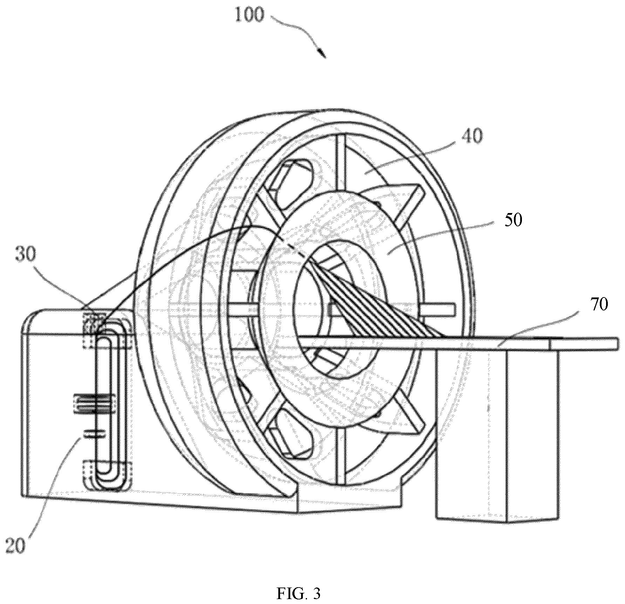

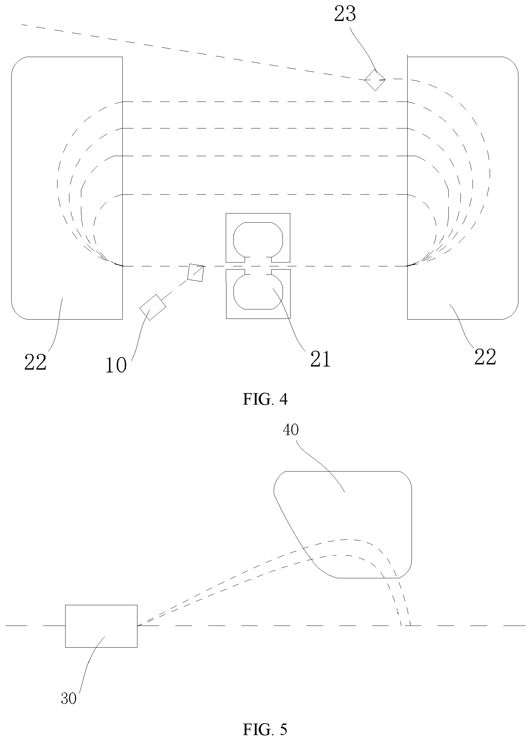

It may be learned from the foregoing that the deflection member 30 can perform first deflection processing on the accelerated electron beam. As a non-limiting example, the deflection member 30 can generate an electromagnetic field and/or an electronic field when in operation, and perform the first deflection processing on the accelerated electron beam by the electromagnetic field and/or the electronic filed. Therefore, referring to FIG. 5, when the apparatus 100 for photon flash treatment is in operation, a magnetic field strength and/or an electronic field strength generated by the deflection member 30 is capable of being adjusted, so as to adjust an angle at which the accelerated electron beam passing through the deflection member 30 enters the deflection mechanism 40. In this way, an objective of changing the target region may be achieved when the apparatus 100 for photon flash treatment is in operation, and a requirement for irradiating a region of interest such as a tumor is met. The accelerated electron beam passing through the deflection member 30 is capable of entering the deflection mechanism 40 at different angles, which means that the accelerated electron beam passing through the deflection member 30 can enter different circumferential positions of the deflection mechanism 40, and/or enter different radial positions of the deflection mechanism 40. As a non-limiting example, referring to FIG. 3, the electron beam processed by the first deflection processing may be emitted to the deflection mechanism 40 at an angle to a plane at which an annular shape of the deflection mechanism 40 is located.

It may be understood that, when the apparatus 100 for photon flash treatment is in operation, a current of the deflection member 30 when powered on may be adjusted to implement adjustment of the magnetic field strength and/or the electronic field strength of the deflection member 30 when powered on. In this way, it may facilitate adjustment of the target region at which the photon beam is converged when the apparatus 100 for photon flash treatment is in operation. The photon beam may be changed by the deflection member 30 to adjust the target region, such as axial electron beam scanning.

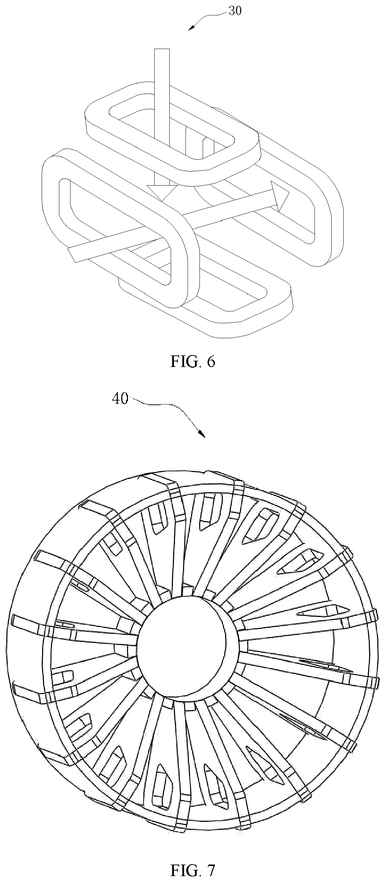

In some embodiments, the deflection member 30 may include either or both of a vector magnet and a deflection resonant cavity. When the deflection member 30 is configured as a vector magnet, referring to FIG. 6, the vector magnet may be alternatively configured as a group of vector magnets that are orthogonal and have adjustable deflection directions. Specifically, the vector magnet may include two pairs of coil assemblies arranged in a vertical direction. When the two pairs of coil assemblies are powered on, two magnetic fields that are vertically arranged in a magnetic field direction may be generated. In this way, when an electron beam passes through the vector magnet in a direction perpendicular to one of the two pairs of coil assemblies, deflection of a motion direction may be performed by a corresponding magnetic field, and the first deflection processing may be performed on the electron beam. Certainly, the two pairs of coil assemblies may control the magnetic field strength by changing the current, so as to perform first deflection processing on the electron beam at different angles when passing through the vector magnet, which is not described herein.

In addition, to implement that a target region (for example, an isocenter position) is adjustable at which a high-energy photon beam is converged when the apparatus 100 for photon flash treatment is in operation, in addition to the foregoing structure of the deflection member 30 with an adjustable magnetic field or an adjustable electronic field when powered on, another deflection member 60 (referring to FIG. 13) may be disposed on a path emitted from the deflection mechanism 40. The deflection member 60 may also generate a magnetic field and/or an electronic field, and a generated magnetic field strength and/or a generated electronic field strength may be adjusted when the deflection member 60 is powered on, so as to adjust the target region. Alternatively, when two deflection members are used to implement adjustment of the target region, the generated magnetic field strength when the deflection member 30 is powered on may not be adjustable. Alternatively, the deflection member 60 disposed on the path emitted from the deflection mechanism 40 may also be specifically configured as a vector magnet or a deflection resonant cavity.

In some embodiments, the deflection member 30 may be separated from the deflection mechanism 40 by a distance in a planar direction of the deflection mechanism 40. The planar direction of the deflection mechanism 40 may include any direction on a plane formed by a longitudinal direction and a width direction of the deflection mechanism 40 or any direction that is parallel to the plane, but is not limited thereto, or may include any direction on any plane at which the deflection mechanism 40 is located or any direction that is parallel to the any plane. For example, as viewed in a main view direction perpendicular to the deflection mechanism 40, there is a distance between the deflection member 30 and the deflection mechanism 40. For example, there is a certain angle between the deflection member 30 and the plane at which the deflection mechanism 40 is located, and the angle may include 0 degree, 10 degrees, 20 degrees, 30 degrees, 45 degrees, and the like. Therefore, the deflection member 30 may be disposed on a side of the deflection mechanism 40, so that other members or devices may be flexibly disposed, thereby saving space in a thickness direction.

In some embodiments, the deflection mechanism 40 may be disposed on a first plane, and the electron beam after the first deflection processing may at least partially pass through the first plane, to the deflection mechanism 40. As a non-limiting example, referring to FIG. 1, the deflection mechanism 40 may be disposed on a first plane (for example, a paper plane in FIG. 1), and the electron beam after the first deflection processing may travel on the first plane to the deflection mechanism 40. In this way, some specific space requirements may be met. For example, referring to FIG. 1, an extension direction of a bed 70 may be perpendicular to the paper plane and perpendicular to the first plane.

In some embodiments, a trajectory of the electron beam leaving the electron beam emitter 9 until the electron beam is converted to the photon beam may be coplanar with the deflection mechanism 40. In this way, it may save space in a thickness direction.

In some embodiments, a trajectory of the electron beam leaving the electron beam generator 10 until the electron beam is converted to the photon beam may be coplanar with the deflection mechanism 40. In this way, it may save space in a thickness direction.

According to the foregoing embodiments, a thickness of the apparatus 100 for photon flash treatment along the extension direction of the bed 70 may be relatively small. When space in a room at which the apparatus 100 for photon flash treatment is located is limited along the extension direction of the bed 70, transport of the bed 70 may be facilitated. In addition, in this way, space is saved, so that other devices (for example, other types of treatment devices, or imaging devices, such as an KV-level orthogonal photon beam, a CT (Computed Tomography), and an MR (Magnetic Resonance)) may be additionally disposed along the extension direction of the bed 70, thereby facilitating a patient to be processed by other devices before, during, or after the flash treatment.

Referring to FIG. 1, FIG. 2, and FIG. 7, in some embodiments, the deflection mechanism 40 may be configured to be distributed around a ring and aggregate the electron beam to a radiation delivery device 50. In this way, the electron beam passing through the deflection mechanism 40 may be converged from different directions within 360 degrees to the radiation delivery device 50 (for example, a same isocenter point, or different discrete positions), thereby further improving a therapeutic effect of the apparatus 100 for photon flash treatment on a tumor when in operation. It should be noted that the deflection mechanism 40 is not limited to a ring structure. One skilled in the art may set a ring angle of the deflection mechanism 40 to any other angle such as 300°, 270°, or 180°. The magnetic field in the deflection mechanism 40 may also be adjustable to achieve beam adjustment (e.g., direction adjustment, e.g., implementation of axial scanning treatment). The deflection mechanism 40 may be a regular shape, or an irregular shape. As examples, the deflection mechanism 40 may be in a ring shape, a pentagon shape, a hexagon shape, an octagon shape or other shape. The deflection mechanism 40 may be entirely annular or partially annular (e.g., merely a partial loop or a broken line). A partially deflection mechanism 40 may be rotated, thereby implementing a desired treatment (e.g., a 360-degree treatment).

Referring to FIG. 7, in some embodiments, the deflection mechanism 40 may include at least one toroidal magnet having a central bore, a primary axis of the toroidal magnet may extend along the central bore, and the toroidal magnet may be configured to receive electron beams at different radial locations or different circumferential locations dependent on a momentum to charge ratio of the electron beam. The toroidal magnet may include a plurality of discrete, substantially planar coils spaced apart and extending radially from the primary axis and configured to produce a magnetic field such that, in use, the electron beam having a component of motion along the primary axis and entering the toroidal magnet at a radial location or a circumferential location, is directed towards the primary axis. The planar coils may be configured to produce a magnetic field that is periodically symmetric about the primary axis, and the at least one toroidal magnet is substantially stationary in use.

As a non-limiting example, the deflection mechanism 40 may include a normal temperature magnet and/or a superconducting magnet, so that when the deflection mechanism 40 is in operation, second deflection processing may be specifically performed on the electron beam by a magnetic field strength, so that the deflected electron beam may be converged to the radiation delivery device 50. It should be noted that when the deflection mechanism 40 includes the normal temperature magnet, a magnetic field value of the normal temperature magnet may be less than 2T. When the deflection mechanism 40 includes the superconducting magnet, a magnetic field value of the superconducting magnet may be higher than that of the normal temperature magnet, and to meet a requirement of the deflection mechanism 40, a low-temperature cooling system generally needs to be provided in the deflection mechanism 40, which may include an Ammonia refrigeration system, a halogenated hydrocarbon refrigeration system, or a refrigeration system with special refrigerant.

In some embodiments, the radiation delivery device 50 may further include either or both of a target assembly 51 and a multi-leaf collimator 52. The target assembly 51 may be configured for bremsstrahlung of the electron beam after the second deflection processing via the deflection mechanism 40 and forming the photon beam. The multi-leaf collimator 52 may be configured to receive the photon beam from the target assembly 51 and be able to shape the photon beam to match a shape of a region of interest in the target region. It should be noted that the target assembly 51 and the multi-leaf collimator 52 may be disposed in a ring structure, or a quantity of the target component 51 and the multi-leaf collimator 52 may be disposed in multiple, and target components 51 and multi-leaf collimators 52 may be disposed along a circumferential direction of the target region at intervals.

It may be learned from the foregoing that the radiation delivery device 50 may be applied to the apparatus 100 for photon flash treatment in the present invention, and an emission direction of the photon beam may be changed. Therefore, a quantity of the radiation delivery device 50 may be set to one, and the radiation delivery device 50 may move around the target region. In this way, movement of the radiation delivery device 50 may be used to change the emission direction of the photon beam when the apparatus 100 for photon flash treatment is in operation. Alternatively, the quantity of the radiation delivery device 50 may be multiple, multiple radiation delivery devices 50 may be arranged at intervals around the target region, and at least one of the multiple radiation delivery devices 50 may be fixed relative to the target region. In this way, different radiation delivery devices 50 may be controlled to operate and generate the photon beam, and the emission direction of the photon beam may be changed when the apparatus 100 for photon flash treatment is in operation.

The radiation delivery device 50 may be mounted on an inner surface of the deflection mechanism 40. The radiation delivery device 50 and the deflection mechanism 40 may be supported by the same supporting mechanism or by separate supporting mechanisms, respectively. The supporting mechanism may include a house that located around the radiation delivery device 50 and the deflection mechanism 40 shown in FIG. 3. When the radiation delivery device 50 can move around the target region, a slide rail may be disposed on the supporting mechanism of the radiation delivery device 50, and the radiation delivery device 50 may move along the slide rail to a specified position according to an instruction of a control host.

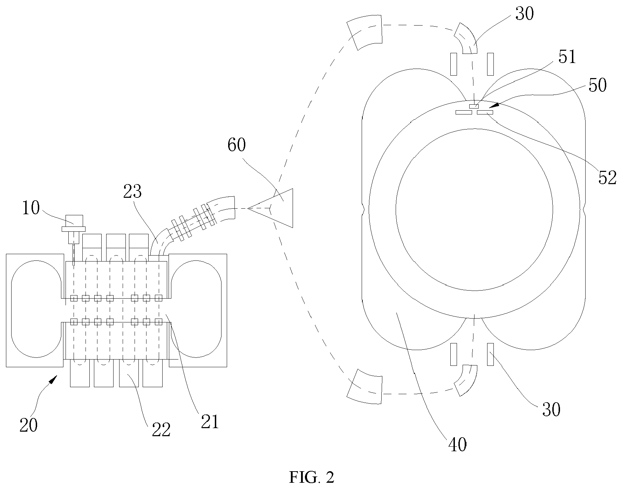

Referring to FIG. 2, in some embodiments, the apparatus 100 for photon flash treatment may include two groups of deflection mechanisms 40, which are arranged at both sides of the target region symmetrically. The apparatus 100 for photon flash treatment may further include a beam splitter 60 configured to direct the electron beam to two groups of deflection mechanisms 40, so that the photon beam generated by the radiation delivery device 50 may cover the target region by 360 degrees. In this way, full coverage of the target region by the photon beam during operation of the apparatus 100 for photon flashover treatment may be implemented, and a flash treatment effect on a patient during the flash treatment may be improved. It should be noted that a specific structure of the beam splitter 60 and an operation principle of how to direct the electron beam to the two groups of deflection mechanisms 40 may be described in a conventional manner.

It should be noted that an electron beam allocated by the beam splitter 60 is not limited to two corresponding groups of deflection mechanisms 40. For one skilled in the art, a quantity of the deflection member 30 may be two. Two deflection members 30 may receive an electron beam allocated by the beam splitter 60, respectively, and perform the first deflection processing on the electron beam by a magnetic field and/or an electric field. The electron beam after the first deflection processing may be directly emitted on the target assembly 51. Alternatively, the electron beam after the first deflection processing may be emitted to the deflection mechanism 40, second deflection processing may be performed by the deflection mechanism 40, and then the electron beam after the second deflection processing may be emitted to the target assembly 51. Thus, a photon beam formed by the target assembly 51 may be enabled to achieve full coverage of the target region with 360 degrees.

It could be understood that a quantity of the radiation delivery device 50 may be two, two radiation delivery devices 50 may be in one-to-one correspondence with the two groups of deflection mechanisms 40. Certainly, a quantity of the radiation delivery device 50 may also be one, and electron beams after the second deflection processing from the two groups of the deflection mechanism 40 may be received by moving the radiation delivery device 50.

Referring to FIG. 3, in some embodiments, the deflection member 30 may be separated from the deflection mechanism 40 by a distance along an axial direction of the deflection mechanism 40. The axial direction may be perpendicular to the planar direction of the deflection mechanism 40. The accelerated electron beam passing through the deflection member 30 is capable of entering the deflection mechanism 40 at different angles, which means that the accelerated electron beam passing through the deflection member 30 can enter different circumferential positions of the deflection mechanism 40, and/or enter different radial positions of the deflection mechanism 40. After the accelerated electron beam passing through the deflection mechanism 40, the electron beam can enter the radiation delivery device 50. The radiation delivery device 50 is configured to convert the electron beam into the photon beam. The photon beam may be emitted towards the target region on the bed 70. As a non-limiting example, there is a certain angle between the deflection member 30 and the axial direction of the deflection mechanism 40.

Referring to FIG. 3, in some embodiments, the radiation delivery device 50 may be in distributed in an annular manner. Alternatively, the radiation delivery device 50 may be disposed in a 360-degree annular structure. In this way, the photon beam converted by the radiation delivery device 50 may irradiate the target region at any angle according to a requirement, thereby further improving efficiency and flexibility of adjustment of the target region formed after the photon beam is converged when the apparatus 100 for photon flash treatment is in operation. It should be noted that the radiation delivery device 50 is not limited to the foregoing 360-degree annular structure. For one skilled in the art, a quantity of the radiation delivery device 50 may be multiple, and the multiple radiation delivery devices 50may be arranged along a circumferential direction around the target region (for example, a circumferential direction of a circular hole enclosed by the deflection mechanism 40) at intervals, so as to meet a requirement for different angles of irradiating to the target region.

As a non-limiting example, the target assembly 51 may include a solid-state target, a rotary target, a liquid metal target, and/or a waterfall particle target. Alternatively, the target assembly 51 may include one or more of metal or alloys such as copper, aluminum, lead, tungsten, copper alloy, aluminum alloy, or lead alloy. The target assembly 51 may be made of an alloy having a higher atomic number, so that energy of the photons excited and released by the electron beam is higher.

In some embodiments, the multi-leaf collimator 52 may be configured to adjust a beam shape of the photon beam from the target assembly 51. In this way, fitness of the apparatus for photon flash treatment to irradiate the region of interest (such as a tumor or another lesion) may be improved while operating, and damage to normal tissue and organs around the tumor may be reduced. It should be noted that the multi-leaf collimator 52 may be specifically disposed in adaptation to the target assembly 51. As an example, the multi-leaf collimator 52 may be distributed in an annular manner, for example, along a circumferential direction of a circular hole enclosed by the deflection mechanism 40. As a non-limiting example, multi-leaf collimators 52 may be arranged along the circumferential direction of the target region at intervals to meet a requirement for different angles of irradiating to the target region. A specific leaf structure of the multi-leaf collimator 52, a control mechanism, and a principle of how the multi-leaf collimator 52 is combined with the target assembly 51 and achieve shaping the photon beam during operation may be implemented by using conventional techniques, which are not described herein.

In addition, in some embodiments, the apparatus 100 for photon flash treatment may further include a treatment head (not shown) that may rotate along a circumference enclosed by the deflection mechanism 40. The treatment head may include the target assembly 51. The electron beam after the second deflection processing of the deflection mechanism 40 may be emitted to the treatment head which is rotated at a position corresponding to the electron beam, the electron beam may further be emitted to the target assembly 51, the photon beam may be formed from the target assembly 51, and the photon beam may be emitted from the treatment head and directed towards the target region. Alternatively, the treatment head may include the multi-leaf collimator 52. In a case that the treatment head includes both the target assembly 51 and the multi-leaf collimator 52, the electron beam after the second deflection processing may be emitted to the treatment head at the corresponding position, the electron beam may further be emitted to the target assembly 51 to form the photon beam, and the photon beam may be shaped by the multi-leaf collimator 52, emitted from the treatment head and directed towards the target region. In a case that the treatment head includes only the multi-leaf collimator 52, the target assembly 51 may be distributed in an annular manner, and the electron beam after the second deflection processing of the deflection mechanism 40 may be emitted to the target assembly 51 to form the photon beam, which in turn is emitted to the treatment head that is rotated to the position corresponding to the photon beam. The multi-leaf collimator 52 in the treatment head may adjust the beam shape of the photon beam, and the adjusted photon beam may be emitted from the treatment head and directed to the target region.

Referring to FIG. 1, the apparatus 100 for photon flash treatment may further include a bed 70. The bed may be disposed through the radiation delivery device 50. The target region formed after the radiation delivery device 50 converges the photon beam that is generated when in operation may be disposed in a region where the bed 70 is located. In this way, when the apparatus 100 for photon flash treatment is in operation, the patient may lie flat on the bed 70 for photon beam irradiation treatment.

In an embodiment, a position of the target region relative to the apparatus for photon flash treatment is capable of being changed during a treatment such as a flash treatment or a volumetric-modulated arc treatment (VMAT). The position of the target region relative to the apparatus for photon flash treatment is changed by adjusting a travel direction of the photon beam and/or adjusting a position of the bed at which the target region is located (e.g., the target region may be changed by moving the bed 70).

In an embodiment, the bed 70 may be rotatable so that the target region is able to be irradiated by the photon beam from any angle.

In addition, an electron transport path in the apparatus 100 for photon flash treatment may be specifically disposed in a closed space, and a vacuum pump 80 may be used to maintain a required vacuum in the electron transport path.

As an example, FIG. 1 shows a case when the deflection mechanism 40 is configured to surround the bed 70 by approximately 360 degrees. In a lateral direction (i.e., a width direction of the bed 70) in FIG. 1, there is a gap in the deflection mechanism 40, so that a part of the electron beam after the first deflection processing performed by the deflection member 30 is not subjected to the second deflection processing. The electron beam after the first deflection processing or the second deflection processing may be emitted to the target assembly 51 to generate the photon beam. However, this is not limited thereto, the deflection mechanism 40 may also be set to completely surround the bed 70 by 360 degrees, surround the bed 70 by less than 360 degrees, or leave one or more gaps at other positions along a circumferential direction, or the like.

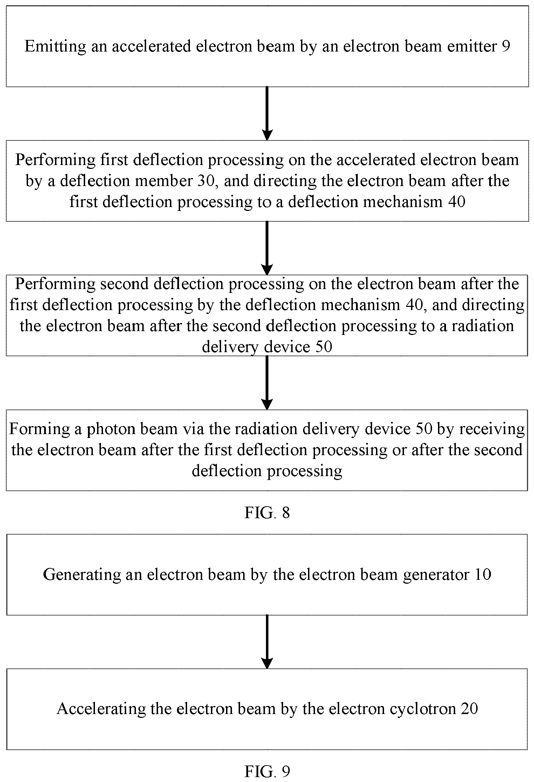

Referring to FIG. 8, the present invention may further provide a method for photon beam formation, including: emitting an accelerated electron beam by an electron beam emitter 9; performing first deflection processing on the accelerated electron beam by a deflection member 30, and directing the electron beam after the first deflection processing to a deflection mechanism 40; performing second deflection processing on the electron beam after the first deflection processing by the deflection mechanism 40, and directing the electron beam after the second deflection processing to a radiation delivery device 50; and forming a photon beam via the radiation delivery device 50 by receiving the electron beam after the first deflection processing or after the second deflection processing. The formed photon beam may be directed to any position. For example, the photon beam may be directed towards the target region that requires treatment or radiation, another photon beam processing device for further processing, or the like. As a non-limiting example, the photon beam may be directed to the target region, thereby allowing the photon beam to reach the target region, and performing radiation treatment, examination, testing, or the like on an object or a target.

Referring to FIG. 9, the electron beam emitter 9 may further include an electron beam generator 10 and an electron cyclotron 20, and emitting an accelerated electron beam by an electron beam emitter 9 may further include: generating an electron beam by the electron beam generator 10, and accelerating the electron beam by the electron cyclotron 20.

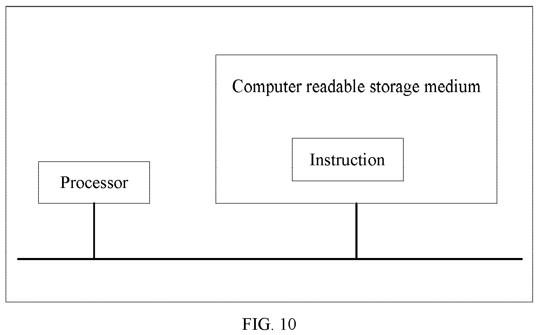

In addition, referring to FIG. 10, the present invention may further provide a computer readable storage medium. An instruction is stored on the computer readable storage medium. When executed by a processor, the instruction implements the following steps: emitting an accelerated electron beam by an electron beam emitter 9; performing first deflection processing on the accelerated electron beam by a deflection member 30, and directing the electron beam after the first deflection processing to a deflection mechanism 40; performing second deflection processing on the electron beam after the first deflection processing by the deflection mechanism 40, and directing the electron beam after the second deflection processing to a radiation delivery device 50; and forming a photon beam via the radiation delivery device 50 by receiving the electron beam after the first deflection processing or after the second deflection processing. The formed photon beam may be directed to any position.

One skilled in the art may understand that all or a part of the processes in the methods in the foregoing embodiments may be implemented by a computer program instructing related hardware. The computer program may be stored in a non-volatile computer readable storage medium. When the computer program is executed, the processes in the foregoing methods embodiments may be included. Any reference to a memory, a database, or another medium used in the embodiments provided in the present invention may include at least one of a non-volatile memory or a volatile memory. The non-volatile memory may include a Read-Only Memory (ROM), a magnetic tape, a floppy disk, a flash memory, an optical memory, a high-density embedded non-volatile memory, a Resistive Random Access Memory (ReRAM), a Magneto resistive Random Access Memory (MRAM), a Ferroelectric Random Access Memory (FRAM), a Phase Change Memory (PCM), a graphene memory, and the like. The volatile memory may include a Random Access Memory (RAM), an external cache, or the like. As an illustration and not a limitation, the RAM may be in multiple forms, such as a Static Random Access Memory (SRAM) or a Dynamic Random Access Memory (DRAM). The databases involved in the embodiments provided in the present invention may include at least one of a relational database or a non-relational database. The non-relational database may include a distributed database based on block chain or the like, which is not limited thereto. The processor in the embodiments provided in the present invention may be a general processor, a central processing unit, a graphics processor, a digital signal processor, a programmable logic device, a data processing logic device based on quantum computing, or the like, which is not limited thereto.

In conclusion, various parts such as the electron cyclotron 20, the deflection member 30, the deflection mechanism 40, and the radiation delivery device 50 may cooperate with each other by using an overall system design and an electronic path design of the apparatus 100 for photon flash treatment, so that a volume of the photon flashover apparatus 100 is greatly reduced and manufacturing costs are reduced while flashover is performed by using a photon beam, and flexible adjustment and control of a target position can be implemented. In this way, it is convenient for a doctor to flashover a patient.

## Claims

1. An apparatus (100) for photon flash treatment, **characterized by** comprising an electron beam emitter (9), a deflection member (30), a deflection mechanism (40), and a radiation delivery device (50), wherein
the electron beam emitter (9) is configured to emit an accelerated electron beam;
the deflection member (30) is configured to be capable of performing first deflection processing on the accelerated electron beam;
the deflection mechanism (40) is configured to perform second deflection processing on the electron beam after the first deflection processing; and
the radiation delivery device (50) is configured to form a photon beam from the electron beam after the first deflection processing or after the second deflection processing.

2. The apparatus (100) of claim 1, wherein the deflection member (30) is separated from the deflection mechanism (40) by a distance in a planar direction of the deflection mechanism (40).

3. The apparatus (100) of claim 1, wherein the deflection member (30) is separated from the deflection mechanism (40) by a distance along an axial direction of the deflection mechanism (40).

4. The apparatus (100) of any one of claims 1 to 3, wherein the deflection mechanism (40) is configured to be distributed around a ring.

5. The apparatus (100) of any one of claims 1 to 4, wherein the radiation delivery device (50) further comprises either or both of a target assembly (51) and a multi-leaf collimator (52), the target assembly (51) is configured to form the photon beam by receiving the electron beam after the first deflection processing or after the second deflection processing, and the multi-leaf collimator (52) is configured to adjust a beam shape of the photon beam from the target assembly (51).

6. The apparatus (100) of any one of claims 1 to 5, wherein the apparatus comprises one radiation delivery device (50) capable of moving around a target region; or
the apparatus comprises a plurality of radiation delivery devices (50) arranged at intervals around the target region, wherein at least one of the plurality of radiation delivery devices (50) is fixed or movable relative to the target region.

7. The apparatus (100) of any one of claims 1 to 6, wherein the electron beam emitter (9) further comprises an electron beam generator (10) and an electron cyclotron (20);
the electron beam generator (10) is configured to generate an electron beam; and
the electron cyclotron (20) is configured to accelerate the electron beam.

8. The apparatus (100) of any one of claims 1 to 7, wherein the apparatus comprises two groups of deflection mechanisms (40), which are arranged at both sides of the target region symmetrically.

9. The apparatus (100) of claim 8, further comprising a beam splitter (60) configured to direct the electron beam to two deflection members (30) through different paths, wherein the two deflection members (30) are corresponding to the two groups of deflection mechanisms (40), respectively.

10. The apparatus (100) of claim 7, wherein the electron cyclotron (20) further comprises an acceleration cavity (21) and two groups of vector magnets (22), and the two groups of vector magnets (22) are arranged at both sides of the acceleration cavity (21).

11. The apparatus (100) of claim 7 or claim 10, wherein the electron cyclotron (20) further comprises a lead-out member (23), the lead-out member (23) is disposed on a cyclic path of the electron beam in the electron cyclotron (20), the lead-out member (23) is configured to lead out the accelerated electron beam, and a position of the lead-out member (23) is capable of being adjusted relative to the electron cyclotron (20).

12. The apparatus (100) of any one of claim 7, claim 10, or claim 11, wherein the electron cyclotron (20) further comprises at least one of a betatron, a petal-shaped accelerator, or a race-track microtron.

13. The apparatus (100) of any one of claims 1 to 12, wherein a generated magnetic field strength or a generated electric field strength of the deflection member (30) is capable of being adjusted when the deflection member (30) is powered on, so that the electron beam after the first deflection processing is capable of entering the deflection mechanism (40) at different angles.

14. A method for photon beam formation, **characterized by** comprising:
emitting an accelerated electron beam by an electron beam emitter (9);
performing first deflection processing on the accelerated electron beam by a deflection member (30), and directing the electron beam after the first deflection processing to a deflection mechanism (40);
performing second deflection processing on the electron beam after the first deflection processing by the deflection mechanism (40), and directing the electron beam after the second deflection processing to a radiation delivery device (50); and
forming a photon beam via the radiation delivery device (50) by receiving the electron beam after the first deflection processing or after the second deflection processing.

15. A computer readable storage medium on which an instruction is stored, **characterized in that** when executed by a processor, the instruction implements the method of claim 14.
